# EUROPÄISCHE PATENTSCHRIFT

(11) **EP 1 671 946 B1**
(45) Veröffentlichungstag und Bekanntmachung des Hinweises auf die Patenterteilung: **26.03.2008**
(21) Anmeldenummer: 05027315.0
(22) Anmeldetag: 14.12.2005
(51) Int. Cl.: C07C 319/04, C07C 319/16, C07C 321/04

(54) **Verwendung von H2S-haltigen Abgasströmen zur Herstellung von schwefelhaltigen Produkten**
Use of H2S-containing exhaust streams for the production of sulfur-containing products
Utilisation du gaz résiduel contenant de hydrogène sulfuré pour la production de produits contentant du soufre

(30) Priorität: 15.12.2004 DE 102004060321
(43) Veröffentlichungstag der Anmeldung: 21.06.2006
(73) Patentinhaber: BASF SE, 67056 Ludwigshafen (DE)
(72) Erfinder: Müller, Christian, 68167 Mannheim (DE); Weber, Markus, Dr., 67061 Ludwigshafen (DE); Stroefer, Eckhard, Dr., 68163 Mannheim (DE)
(74) Vertreter: Isenbruck, Günter

(56) Entgegenhaltungen:
- EP-A- 0 122 654

## Beschreibung

Die vorliegende Erfindung betrifft ein Verfahren zur Herstellung von Thiolen, Thioethern und Disulfiden durch Umsetzung von Olefinen mit Schwefelwasserstoff in Gegenwart von Wasser, Kohlendioxid und gegebenenfalls Sauerstoff.

Alkylthiole mit 10 bis 30 Kohlenstoffatomen sind bekannte Verbindungen. Alkylthiole oder Gemische dieser Verbindungen werden üblicherweise durch sauer katalysierte elektrophile Addition von Schwefelwasserstoff (H₂S) an Olefine erhalten. Gemäß der Markownikoff-Regel entsteht dabei aus Olefinen, die an ihrer Doppelbindung mindestens drei Alkylsubstituenten tragen, ein tertiäres Thiol und aus linearen Olefinen ein sekundäres Thiol.

Sekundäre Thiole finden Anwendung als Duftstoffe, als Komponenten in Schmiermittel-Zubereitungen und als Härter für Epoxid-Harze. Des Weiteren werden sie vorteilhaft als Zwischenprodukte bei der Herstellung von oberflächenaktiven Verbindungen verwendet. EP-A-0 122 654 beschreibt die Herstellung sekundärer Thiole.

Tertiäre Thiole werden als Molmassenregulator bei Polymerisationen verwendet, insbesondere für radikalische Polymerisationen vinylischer Monomere wie beispielsweise Polymerisation von Butadien, Styrol, carboxylierten Styrol, Acrylsäure, Acrylnitril, Acrylester, Vinylether oder ihren Gemischen.

Ullmann's Encyclopedia of Industrial Chemistry, 6th edition, 2000 Electronic Release (Wiley VCH Verlag GmbH, Weinheim, Deutschland, 2000) gibt unter dem Stichwort "Thiols and Organic Sulfides", Punkt 1.3., "Production of Aliphatic Thiols", unter Punkt 1.3.2. "From Alkenes" einen Überblick über bekannte Methoden zur Herstellung von Alkylthiolen. Gängige Olefingemische, die mit Schwefelwasserstoff an einem sauren Katalysator zu tert.-Dodecylthiol umgesetzt werden, sind trimerisiertes iso-Buten und tetramerisiertes Propen. Beides sind bekannte Gemische stark verzweigter Alkene, die früher in relativ großem Umfang zur Herstellung von anionischen Tensiden eingesetzt wurden. Bei der Wahl des Katalysators ist darauf zu achten, dass das eingesetzte Olefin oder Olefingemisch am gewählten Katalysator keine zu hohe Polymerisationsneigung aufweist, da dieser, falls sich Oligomere oder Polymere auf ihm ansammeln, deaktiviert wird, was einen häufigeren Katalysatorwechsel nötig machen und dadurch die Wirtschaftlichkeit des Verfahrens beeinträchtigen kann.

P. Bapseres, Chim. Ind. (Paris) 90 (1963), S. 358 ff., offenbart ein Verfahren zur Herstellung von tert.-Dodecylthiol aus tetramerem Propen und Schwefelwasserstoff bei -40 °C in Gegenwart von Bortrifluorid oder Aluminiumtrichlorid als Katalysator. J. F. Franz und K. I. Glass, Chem. Eng. Prog. 59 (Heft 7), 1963, Seite 68 ff., lehren ein Verfahren zur Herstellung von tert.-Dodecylthiol aus tetramerem Propen und Schwefelwasserstoff bei 49 °C bis 71 °C in Gegenwart von Bortrifluorid als Katalysator.

EP 0122654 offenbart ein Verfahren zur Herstellung von sekundären Thiolen mit 10 bis 22 Kohlenstoffatomen bei einer Temperatur von 40 bis 140 °C und einem Druck von 10 bis 100 bar ebenfalls in Gegenwart eines Zeoliths als Katalysator.

GB 625 646 beschreibt ein Verfahren zur Schwefelwasserstoffaddition an trimeres iso-Buten mit Ton als Katalysator, der wahlweise mit Schwefel- oder Phosphorsäure aktiviert wird.

US 3,214,386 lehrt die Verwendung einer Mischung aus Phosphorsäure, Bortrifluorid und einem Alkohol mit 1 bis 5 Kohlenstoffatomen als Katalysator bei der Addition von Schwefelwasserstoff an Dopperlbindungen von Olefinen.

Bei dem aus dem Stand der Technik bekannten Verfahren zur Umsetzung von Schwefelwasserstoff mit Olefinen werden Feststoffsäuren wie Ionenaustauscherharze (IOT) und Zeolithe als Katalysatoren eingesetzt. Im Allgemeinen muss bei den Verfahren des Standes der Technik der Schwefelwasserstoff vor der Umsetzung durch physikalische und/oder chemische Methoden gereinigt werden.

Nachteile des Einsatzes von festen Verbindungen als Katalysator sind:
- Transportvorgänge innerhalb des Feststoffs (Pore) stellen oftmals den geschwindigkeitsbestimmenden Reaktionsschritt dar. Große Reaktoren mit großen Katalysatormengen sind die Folge.
- Ionenaustauscherharze sind temperaturempfindlich und verlangen aufgrund der exothermen Reaktion nach einer steten Wärmeabfuhr von den reaktiven Zentren. Daraus folgt teure Parallelbauweise, beispielsweise als Rohrbündelreaktoren.
- lonenaustauscherharze sind mechanisch empfindlich und können ohne nennenswerten Abrieb nur in Festbetten eingesetzt werden.
- Zeolithe verlieren sehr schnell ihre saure Wirkung und müssen aufwendig außerhalb des Reaktors, beispielsweise bei 500 °C, regeneriert werden.
- Zeolithpulver bestehen zum Teil aus sehr kleinen Partikeln im Bereich weniger Mikrometer, die sehr aufwendig aus der Reaktionsmischung abgetrennt werden müssen. Findet die Abtrennung außerhalb des Reaktors statt, so muss außerdem der Katalysator zurückgeführt werden.
- Festbetten aus Formkörpern auf Basis Zeolithpulver werden während des Durchströmens mit z.B. Flüssigkeiten mechanisch zerrieben (Nachteil kleiner Partikel siehe oben).

Bei den bekannten Verfahren zur Umsetzung von Olefinen mit Schwefelwasserstoff werden die in Mischung mit Schwefelwasserstoff vorhandenen Nebenkomponenten, wie Wasser und/oder Kohlendioxid, vor der Umsetzung im Allgemeinen durch übliche vorangestellte physikalische und/oder chemische Verfahren abgetrennt.

Technisch fällt Schwefelwasserstoff in riesigen Mengen bei der Erdölentschwefelung sowie bei der Förderung gewisser "saurer" Erdgase an; darüber hinaus kann Schwefelwasserstoff aus Heizgas, Kokereigas und anderen aus Kohle hergestellten Gasen (Wassergas, Synthesegas) gewonnen werden (aus "Hollemann Wiberg, Lehrbuch der Anorganischen Chemie, deGruyter 1985, Seite 486).

Geeignete Methoden zur Abtrennung von Schwefelwasserstoff aus Mischgasströmen sind beispielsweise die Adsorption in einer Ethanolamin-Lösung (Girbotol-Verfahren) oder chemische Umwandlung (Claus-Verfahren). Beim Claus-Verfahren handelt es sich um einen wichtigen Prozess zur Gewinnung von Schwefel durch Oxidation von Schwefelwasserstoff durch SO₂. Ein Teil des hierbei gewonnenen Schwefels wird anschließend in der Synthese von Schwefelwasserstoff eingesetzt.

US 4,093,701 offenbart ein Verfahren zur selektiven Entfernung von Schwefelwasserstoff aus einem Abgasgemisch, das neben Schwefelwasserstoff Kohlendioxid beinhaltet, durch Umsetzung mit einer wässrigen Alkanolamin-Lösung.

DE 27 54 118 offenbart ein Verfahren zur Entfernung von Schwefelwasserstoff aus Abgasen durch Umsetzung von Schwefelwasserstoff und Kohlendioxid enthaltenden Abgasströmen mit Wasserstoff über einem Katalysator bei 180 bis 450 °C.

Aufgabe der vorliegenden Erfindung ist es, ein Verfahren zur Herstellung von Thiolen, Thioethern und Disulfiden durch Umsetzung von Olefinen mit Schwefelwasserstoff und gegebenenfalls Sauerstoff bereitzustellen, in dem Mischgasströme, die Schwefelwasserstoff enthalten, eingesetzt werden können, ohne dass vor der Umsetzung der Schwefelwasserstoff durch physikalische und/oder chemische Verfahren abgetrennt und/oder gereinigt werden muss.

Diese Aufgabe wird gelöst durch ein Verfahren zur Herstellung von schwefelhaltigen Verbindungen der allgemeinen Formel (I) 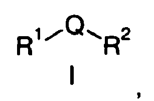 worin Q, R¹ und R² unabhängig voneinander die folgenden Bedeutungen haben:
- Q:: -S- oder -S-S-,
- R¹:: Wasserstoff oder gesättigter oder ungesättigter, linearer oder verzweigter C₁₋C₃₀-Alkylrest,
- R²:: Wasserstoff oder gesättigter oder ungesättigter, linearer oder verzweigter C₁₋C₃₀-Alkylrest,
wobei R¹ und R² nicht gleichzeitig Wasserstoff sind, durch Umsetzung eines Schwefelwasserstoff und gegebenenfalls Sauerstoff enthaltenden Mischgasstroms mit linearen oder verzweigten C₁-C₃₀-Olefinen, wobei die Umsetzung in Gegenwart von Wasser und Kohlendioxid bei einem Druck von 2 bis 325 bar durchgeführt wird.

Das erfindungsgemäße Verfahren wird in Gegenwart von Kohlendioxid und Wasser durchgeführt. In einer bevorzugten Ausführungsform sind Wasser und Kohlendioxid bereits in dem Mischgasstrom enthalten, d.h. Wasser und Kohlendioxid werden dem Reaktionsgemisch folglich nicht zusätzlich zugesetzt.

Durch das erfindungsgemäße Verfahren werden schwefelhaltige Verbindungen der allgemeinen Formel (I) 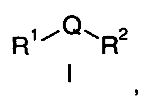 worin Q, R¹ und R² unabhängig voneinander die folgenden Bedeutungen haben:
- Q:: -S- oder -S-S-,
- R¹:: Wasserstoff oder gesättigter oder ungesättigter, linearer oder verzweigter C₁₋C₃₀-Alkylrest, bevorzugt C₈-C₂₂-Alkylrest, besonders bevorzugt C₁₀-C₂₂₋Alkylrest, ganz besonders bevorzugt C₁₀-C₁₆-Alkylrest,
- R²:: Wasserstoff oder gesättigter oder ungesättigter, linearer oder verzweigter C₁₋C₃₀-Alkylrest, bevorzugt C₈-C₂₂-Alkylrest, besonders bevorzugt C₁₀-C₂₂₋Alkylrest, ganz besonders bevorzugt C₁₀-C₁₆-Alkylrest,
wobei R¹ und R² nicht gleichzeitig Wasserstoff sind, erhalten.

Die Reste R¹ und R² können zusätzlich substituiert sein. Geeignete Substituenten sind beispielsweise gesättigte, ungesättigte und/oder aromatische Reste mit 1 bis 20 Kohlenstoffatomen, oder funktionelle Gruppen wie Halogene, Hydroxy-, Aldehyd-, Keto-, Carboxy-, Amid-, Imid- oder Estergruppen. Auch die Kohlenstoffsubstituenten mit 1 bis 20 Kohlenstoffatomen können mit beispielsweise den genannten funktionellen Gruppen substituiert sein.

Q hat die Bedeutung -S- oder -S-S-. Ist in dem erfindungsgemäß einsetzbaren Mischgas neben Schwefelwasserstoff, Kohlendioxid und Wasser kein Sauerstoff anwesend, so bedeutet Q -S-, d.h., dass Thiole oder Thioether gebildet werden. Ist in dem erfindungsgemäßen Mischgas neben Schwefelwasserstoff, Kohlendioxid und Wasser Sauerstoff anwesend, so hat Q die Bedeutung -S-S-, d.h. in dem erfindungsgemäßen Verfahren werden Disulfide gebildet. In Anwesenheit von Sauerstoff in dem erfindungsgemäß einsetzbaren Mischgas können neben Verbindungen, in denen Q -S-S- bedeutet, auch Verbindungen, in denen Q -S- bedeutet, vorliegen.

In einer ganz besonders bevorzugten Ausführungsform bedeuten R¹ und R² in der allgemeinen Formel (I) unabhängig voneinander Wasserstoff oder einen gesättigten Alkylrest mit 12 Kohlenstoffatomen, wobei R¹ und R² nicht gleichzeitig Wasserstoff bedeuten.

In dem Verfahren gemäß der vorliegenden Erfindung werden C₁-C₃₀-Olefine, bevorzugt C₈-C₂₂-Olefine, besonders bevorzugt C₁₀-C₂₂-Olefine, ganz besonders bevorzugt C₁₀₋C₁₆-Olefine, insbesondere bevorzugt C₁₂-Olefine eingesetzt. Es können Mischungen von Olefinen mit unterschiedlicher Kohlenstoffanzahl und/oder unterschiedlichem Substitutionsmuster oder einheitliche Olefine eingesetzt werden. Diese Olefine bzw. Mischungen von Olefinen können beispielsweise durch Cracken von Paraffinwachs oder Oligomerisierung von Ethen erhalten werden. Die resultierenden Oligomerisierungsprodukte weisen größtenteils eine lineare Struktur auf, während Olefine, die durch Oligomerisierung von Propen und/oder Butenen erhalten werden, verzweigt sind.

Die erfindungsgemäß einsetzbaren Olefine können pro Molekül eine oder mehrere Doppelbindung(en) aufweisen. Bevorzugt werden Olefine eingesetzt, die pro Molekül eine Doppelbindung aufweisen, so genannte Mono-Olefine.

Die in dem erfindungsgemäßen Verfahren einsetzbaren Olefine können zum einen α-Olefine mit einer endständigen Doppelbindung sein, zum anderen kann die Doppelbindung auch intern in dem Kohlenwasserstoff vorliegen.

Solche linearen internen Olefine können beispielsweise durch Chlorierung - Dechlorierung von Paraffinen, durch Paraffin-Dehydrierung und durch α-Olefin-Isomerisierung erhalten werden. Bedingt durch das Herstellungsverfahren können die einsetzbaren Olefine bzw. Olefingemische Verunreinigungen, beispielsweise aromatische Verbindungen und/oder gesättigte Kohlenwasserstoffe, zu einem Anteil von bis zu 3 Gew.-% enthalten. Diese Verunreinigungen beeinflussen das erfindungsgemäße Verfahren nicht.

Die Olefine können linear sein oder ein oder mehrere Alkylsubstituenten entlang der Kohlenstoffhauptkette aufweisen. Werden in dem erfindungsgemäßen Verfahren Olefine eingesetzt, deren Doppelbindung endständig ist oder zwei Substituenten aufweist, so werden sekundäre Schwefelverbindungen erhalten, d.h., dass das die -S-, -S-S-, -SH oder -S-S-H-Funktionalität tragende Kohlenstoffatom mit zwei weiteren Kohlenstoffatomen verbunden ist. Werden Olefine eingesetzt, die an der Doppelbindung wenigstens drei Substituenten tragen, so werden tertiäre Schwefelverbindungen erhalten, d.h., dass das die -S-, -S-S-, -SH oder -S-S-H-Funktionalität tragende Kohlenstoffatom mit drei weiteren Kohlenstoffatomen verbunden ist. Durch das erfindungsgemäße Verfahren werden bevorzugt tertiäre Alkylthiole hergestellt.

Ganz besonders bevorzugt werden in dem erfindungsgemäßen Verfahren Olefine mit 12 Kohlenstoffatomen eingesetzt. Durch das Herstellungsverfahren bedingt kann die eingesetzte Olefinkomponente Verunreinigungen durch Olefine mit einer von 12 abweichenden Kohlenstoffanzahl bis zu einem Anteil von 5 Gew.-%, bevorzugt von 3 Gew.-% aufweisen.

Die in dem erfindungsgemäßen Verfahren einsetzbaren Dodecene entsprechen insbesondere bevorzugt einem oder mehreren Olefinen, die von den folgenden Verbindungen abgeleitet sind.

Olefine, die abgleitet sind von n-Dodecan (I)

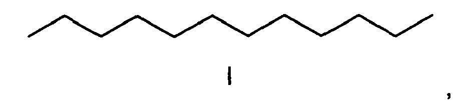

Olefine, die abgleitet sind von 5-Methyl-n-undecan (II)

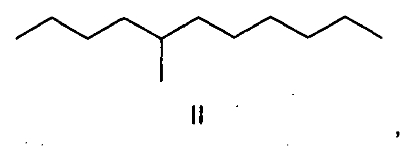

Olefine, die abgeleitet sind von 4-Ethyl-n-decan (III)

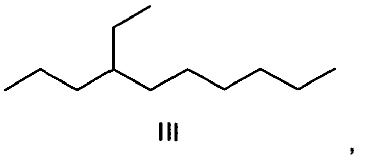

Olefine, die abgeleitet sind von 5,6-di-Methyl-n-decan (IV)

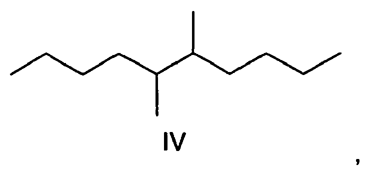

Olefine, die abgeleitet sind von 5-Ethyl-6-methyl-n-nonan (V)

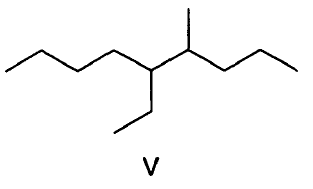

und Olefine, die abgleitet sind von 4,5-di-Ethyl-n-octan (VI)

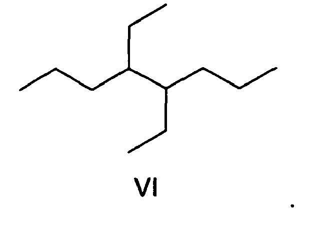

Unter "abgeleitetem Olefin" ist ein Olefin zu verstehen, das von dem betreffenden Alkan formal durch Dehydrierung, also Entfernung zweier Wasserstoffatome von benachbarten Kohlenstoffatomen unter Ausbildung einer Doppelbindung zwischen diesen Kohlenstoffatomen entsteht, wobei jedoch das Kohlenstoffgerüst unverändert bleibt. Es ist weder möglich noch notwendig, die Lage der Doppelbindung genau anzugeben, da sowohl bei üblichen Methoden der Herstellung solcher Gemische (wie beispielsweise unten angegeben) als auch bei der Umsetzung von Olefinen mit Schwefelwasserstoff die Doppelbindung entlang der Kohlenstoffkette wandert. Die Addition von Schwefelwasserstoff an Olefine in Gegenwart von sauren Katalysatoren ist eine reversible Reaktion, wobei jedoch die Doppelbindung sich anders ausbilden kann als sie vorher in der Kohlenstoffkette lag. Insgesamt stellen sich die Lage der Doppelbindung in den Kohlenstoffgerüsten und damit auch die Position der Thiolgruppe gemäß den angewendeten Bedingungen thermodynamisch oder auch kinetisch kontrolliert ein.

Bevorzugt wird in dem erfindungsgemäßen Verfahren ein Kohlenwasserstoffgemisch eingesetzt, welches mindestens 10 Gew.-%, vorzugsweise mindestens 12 Gew.-% und in besonders bevorzugter Weise mindestens 13 Gew.-%, sowie höchstens 18 Gew.-%, vorzugsweise höchstens 16 Gew.-% und in besonders bevorzugter Weise höchstens 15 Gew.-% von n-Dodecan (I) abgeleitetes Olefin,

mindestens 25 Gew.-%, vorzugsweise mindestens 30 Gew.-% und in besonders bevorzugter Weise mindestens 34 Gew.-%, sowie höchstens 40 Gew.-%, vorzugsweise höchstens 38 Gew.-% und in besonders bevorzugter Weise höchstens 36 Gew.-% von 5-Methyl-n-undecan (II) abgeleitetes Olefin,

mindestens 25 Gew.-%, vorzugsweise mindestens 30 Gew.-% und in besonders bevorzugter Weise mindestens 32 Gew.-%, sowie höchstens 40 Gew.-%, vorzugsweise höchstens 38 Gew.-% und in besonders bevorzugter Wiese höchstens 34 Gew.-% von 4-Ethyl-n-decan (III) abgeleitetes Olefin,

mindestens 2 Gew.-%, vorzugsweise mindestens 4 Gew.-% und in besonders bevorzugter Weise mindestens 5 Gew.-%, sowie höchstens 8 Gew.-%, vorzugsweise höchstens 7 Gew.-% von 5,6-di-Methyl-n-decan (IV) abgeleitetes Olefin,

mindestens 5 Gew.-%, vorzugsweise mindestens 6 Gew.-% und in besonders bevorzugter Weise mindestens 8 Gew.-%, sowie höchstens 12 Gew.-%, vorzugsweise höchstens 10 Gew.-% von 5-Ethyl-6-methyl-n-nonan (V) abgeleitetes Olefin, mindestens 1 Gew.-%, vorzugsweise mindestens 2 Gew.-%, sowie höchstens 5 Gew.-%, vorzugsweise höchstens 4 Gew.-% und in besonders bevorzugter Weise höchstens 3.5 Gew.-% von 4,5-Di-ethyl-n-octan (VI) abgeleitetes Olefin

und höchstens 5 Gew.-%, vorzugsweise höchstens 3 Gew.-% anderer Kohlenwasserstoffe, mit der Maßgabe, dass die Summe der Anteile der Komponenten 100 Gew.-% beträgt, enthält.

Ganz besonders bevorzugt wird durch das erfindungsgemäße Verfahren aus Dodecen und Schwefelwasserstoff tert.-Dodecylthiol hergestellt. Dabei können neben tertiären Dodecylthiolen auch primäre und/oder sekundäre Dodecylthiole vorliegen.

Die Konfiguration (cis- oder trans-Konfigurationsisomerie) der eingesetzten Olefine ist nicht erheblich. Im Allgemeinen werden die Olefine in der Konfiguration (oder in Form des Gemisches von Konfigurationsisomeren) verwendet, in der sie erzeugt werden, was meistens der thermodynamisch vorgegebenen relativen Stabilität der Isomeren entspricht.

Die Umsetzung von Olefinen (VIII) mit Schwefelwasserstoff zu Thiolen (IX) verläuft im Allgemeinen nach dem folgenden Reaktionsschema

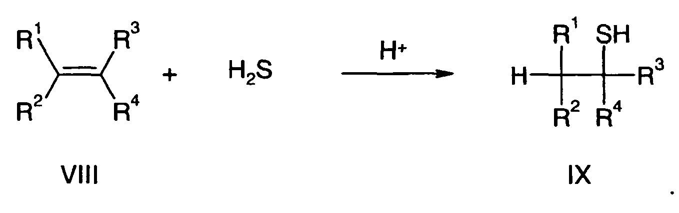

Das während der Reaktion gebildete Thiol (IX) kann mit einem weiteren Äquivalent Olefin (VIII) zu dem entsprechenden Thioether (X) reagieren

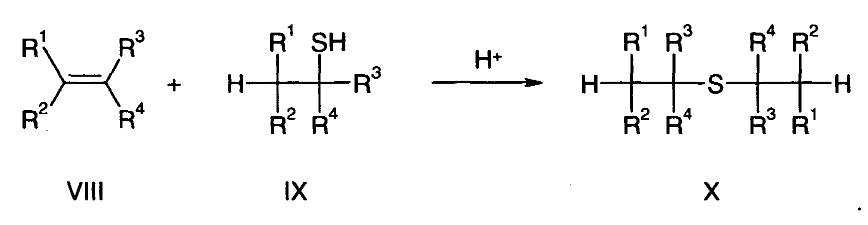

Enthält der erfindungsgemäß einsetzbare Mischgasstrom neben Schwefelwasserstoff, Kohlendioxid und Wasser auch Sauerstoff, so reagiert das gebildete Thiol (IX) nach folgendem Schema mit einem halben Molekül Sauerstoff und einem Molekül Schwefelwasserstoff zu Disulfid (XI) 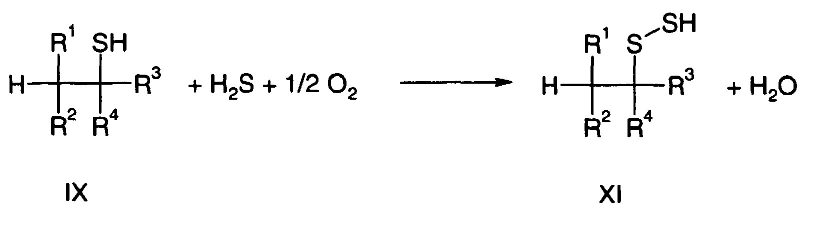 bzw. es reagieren zwei Moleküle Thiol (IX) mit einem halben Molekül Sauerstoff zu Disulfid (XII)

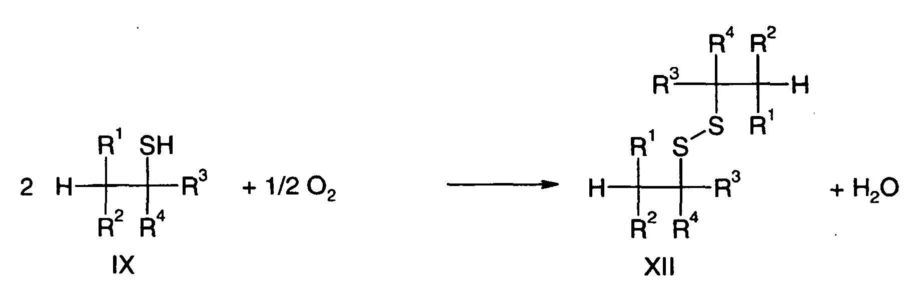

Das erfindungsgemäße Verfahren zeichnet sich dadurch aus, dass in Abhängigkeit der Zusammensetzung des eingesetzten Mischgases die oben genannten Schwefel enthaltenden Verbindungen Thiole, Thioether und/oder Disulfide mit einem oder zwei Alkylsubstituenten erhalten werden können. Die entsprechenden Disulfide werden gebildet, wenn das eingesetzte Mischgas neben Schwefelwasserstoff, Kohlendioxid und Wasser auch Sauerstoff enthält. In Gegenwart von Sauerstoff handelt es sich bei dem erfindungsgemäßen Verfahren um ein Verfahren zur Herstellung von Disulfiden in einem Reaktor ohne Isolierung und/oder Aufreinigung von Zwischenprodukten.

Das erfindungsgemäße Verfahren kann kontinuierlich, semikontinuierlich oder diskontinuierlich durchgeführt werden. In einer Ausführungsform kann das erfindungsgemäße Verfahren kontinuierlich betrieben werden, d.h., dass das Produkt kontinuierlich abgeführt wird, und die Substrate gemäß ihrem Verbrauch kontinuierlich zugeführt werden, so dass im Mittel im Reaktionsgefäß konstante Konzentrationen aller vorhandenen Stoffe vorliegen. Für die kontinuierliche Verfahrensweise geeignete Reaktionsgefäße sind dem Fachmann bekannt. Beispielhaft sind Rohrreaktoren, Rührreaktoren, Umlaufreaktoren genannt.

In einer weiteren Ausführungsform kann das erfindungsgemäße Verfahren semikontinuierlich durchgeführt werden, d.h. dass die Substrate gemischt werden, die Reaktion gestartet wird, und entstehende Produkte kontinuierlich, beispielsweise durch Destillation, abgeführt werden.

Bei der diskontinuierlichen Verfahrensweise werden die beteiligten Substrate gemischt, die Reaktion wird gestartet, und nach Beendigung der Reaktion wird die Reaktionsmischung als Ganzes durch geeignete Methoden, beispielsweise Destillation, aufgearbeitet.

Aus Kohlendioxid und Wasser, welche in dem erfindungsgemäß einsetzbaren Mischgas enthalten sind, bildet sich zu Beginn der Umsetzung Kohlensäure

H₂O (I) + CO₂ (aq.) → H₂CO₃ (aq.).

Es wurde gefunden, dass die unter Druck in dem Mischgas, gegebenenfalls unter Zugabe von weiterem Wasser, aus Kohlendioxid und Wasser gebildete Kohlensäure H₂CO₃ als Katalysator zur Herstellung von Thiolen, Sulfiden oder Disulfiden aus den im Mischgas vorhandenen Olefinen und Schwefelwasserstoff geeignet ist.

Vorteilhaft an dem Verfahren gemäß der vorliegenden Erfindung ist, dass sich die katalytisch aktive Spezies aus Verbindungen bildet, die bereits in dem einzusetzenden Substrat, dem Mischgas, enthalten sind. Es müssen folglich keine weiteren Katalysatoren, feste oder flüssige, in die erfindungsgemäße Reaktionsmischung eingebracht werden. Daraus resultieren eine stark vereinfachte Reaktionsführung und die Vermeidung der erwähnten Nachteile, die bei Verwendung von festen sauren Katalysatoren auftreten.

Die erfindungsgemäße Reaktion wird in einer bevorzugten Ausführungsform bei einem Druck von 10 bis 100 bar, besonders bevorzugt 15 bis 50 bar durchgeführt.

Das erfindungsgemäße Verfahren wird in einer bevorzugten Ausführungsform bei einer Temperatur von 0 bis 100 °C, besonders bevorzugt 5 bis 80 °C, ganz besonders bevorzugt bei 30 bis 60 °C durchgeführt.

Je nach Wassergehalt der Einsatzstoffe wird der Reaktionsmischung gegebenenfalls zusätzliches Wasser zugesetzt. In einer bevorzugten Ausführungsform liegt in der Reaktionsmischung Wasser zu 0,1 bis 50 Gew.-%, besonders bevorzugt zu 10 bis 40 Gew.-%, ganz besonders bevorzugt zu 20 bis 30 Gew.-% vor.

Nachdem in dem erfindungsgemäßen Verfahren ein Umsatz von im Allgemeinen > 85%, bevorzugt > 90%, besonders bevorzugt > 95%, erreicht worden ist, wird die Reaktionsmischung entspannt, d.h. der Druck in dem Reaktionsgefäß wird auf Normaldruck abgesenkt, wodurch nicht reagierte Gasanteile desorbiert werden. Gegebenfalls kann die Reaktionsmischung zur Freisetzung der noch vorhandenen Gasanteile auf bis zu 100 °C aufgeheizt werden.

Die durch das erfindungsgemäße Verfahren hergestellten Schwefel enthaltenden Verbindungen können gegebenenfalls nach Beendigung der Reaktion nach allen dem Fachmann bekannten Methoden aufgearbeitet und/oder gereinigt werden. Beispielsweise sind Phasentrennung, Extraktion, Destillation u.a. genannt.

Das erfindungsgemäße Verfahren kann in An- oder in Abwesenheit eines weiteren Lösungsmittels neben Wasser durchgeführt werden. Bevorzugt wird das Verfahren in Anwesenheit von Wasser und Abwesenheit weiterer Lösungsmittel durchgeführt.

Enthält das in das erfindungsgemäße Verfahren eingesetzte Mischgas neben Schwefelwasserstoff, Kohlendioxid und Wasser auch Sauerstoff, so werden Disulfide der Formel (XI) und/oder (XII) gebildet. Der in dem Mischgasstrom enthaltene Sauerstoff kann aus der Reaktion, aus dem der Mischgasstrom herrührt, stammen. Es ist erfindungsgemäß auch möglich, dass Sauerstoff dem Mischgasstrom vor Einleiten in das Reaktionsgefäß zugesetzt wird. Dabei wird der Sauerstoff oder ein Sauerstoff enthaltendes Gas in dem Maße zugesetzt, dass er für die angestrebte chemische Reaktion in der richtigen Menge vorliegt. Für das erfindungsgemäße Verfahren geeignete Mengen an Sauerstoff sind beispielsweise 0,1 bis 10 Gew.-%, bevorzugt 2 bis 6 Gew.-%, besonders bevorzugt 3 bis 4 Gew.-%.

Das in das erfindungsgemäße Verfahren einsetzbare Mischgas stammt in einer bevorzugten Ausführungsform aus dem Abgasstrom eines chemischen Verfahrens. Dieser Abgasstrom kann aus allen chemischen Reaktionen stammen, in denen im Abgasstrom neben Schwefelwasserstoff auch Kohlendioxid und gegebenenfalls Sauerstoff vorliegen. Beispiele für geeignete chemische Verfahren sind Verfahren zur Herstellung von Synthesegas oder solche Verfahren, bei denen Heizgas, Kokereigas oder andere aus Kohle hergestellten Gase entstehen.

Das Abgas, welches das Reaktionsgefäß nach der Reaktion verlässt, hat aufgrund des Verbrauchs an Schwefelwasserstoff zur Herstellung der Schwefel enthaltenden Verbindungen einen geringeren Anteil an Schwefelwasserstoff als das Mischgas, welches als Substrat in die Reaktion eingesetzt worden ist. Je nach stöchiometrischem Verhältnis n(H₂S)/n(Doppelbindungen) von Schwefelwasserstoff zu Olefinen in der Reaktionsmischung kann dieses Abgas völlig von Schwefelwasserstoff befreit werden.

Schwefelwasserstofffrei bedeutet im Sinne der vorliegenden Anmeldung einen Gehalt an Schwefelwasserstoff im Abgas des erfindungsgemäßen Verfahrens von ≤ 0,5%, bevorzugt ≤ 0,1% , besonders bevorzugt ≤ 0,0001 %, ganz besonders bevorzugt ≤ 0,00002%.

Somit eignet sich das Verfahren gemäß der vorliegenden Erfindung auch zu Reinigung von Schwefelwasserstoff enthaltenden Mischgasströmen, um Schwefelwasserstoff freie Abgase zu erhalten.

Vorteile des erfindungsgemäßen Verfahrens sind:
- Bei der Herstellung von Schwefel enthaltenden Verbindungen muss der eingesetzte Schwefelwasserstoff nicht vorher aufwendig mit physikalischen und/oder chemischen Verfahren gereinigt werden.
- Durch die Verwendung des in dem eingesetzten Mischgasstrom enthaltenen Kohlendioxids als katalytisch aktive Komponente in dem erfindungsgemäßen Verfahren muss kein weiterer Katalysator zugesetzt werden, wodurch die zuvor schon genannten Nachteile, etwa bei der Verwendung eines festen sauren Katalysators, umgangen werden können.
- Das erfindungsgemäße Verfahren kann zur Reinigung Schwefelwasserstoff enthaltender Mischgasströme, die in großem Maßstab bei chemischen Prozessen anfallen, eingesetzt werden.

### Beispiele:

### Beispiel 1

Es werden 12 g Wasser und 40 g Dodecen über einen Trichter in die Anlage eingefüllt. Nach Verschließen der Anlage wird die Reaktionsmischpumpe in Betrieb genommen (Drehzahl: 2800 min⁻¹) und der gesamte Umpumpkreislauf, der inklusive Pumpenkopf in Doppelmantelausführung gefertigt ist, auf Starttemperatur von 30 °C gebracht. Gestartet wird die Reaktion durch stufenweises Aufpressen von zuerst 10 bar Kohlendioxid und anschließend 20 bar Schwefelwasserstoffgas auf einen Gesamtdruck von ca. 31 bar. Während des Experiments bleibt die H2S-Zuleitung des Reaktorsystems geöffnet, so dass die abreagierte Schwefelwasserstoffmenge in das Reaktionssystem nachgeliefert wird. Während der nächsten 2 Stunden wird gleichmäßig auf 60 °C erwärmt. Nach 3 weiteren Stunden werden mittels Druckabsenkung am Entnahmehahn und zusätzliches Aufheizen auf 100 °C alle gelösten Gasbestandteile aus der zweiphasigen Flüssigprobe entfernt. Die Zusammensetzung der organischen Flüssigphase (obere Phase) wird mittels Gaschromatographie analysiert, wobei zwischen Edukt (Olefin), Hauptprodukt (Thiol) und Nebenkomponente (Thioether) unterschieden werden kann.

**Tabelle 1**

| | GC-Analysen-Ergebnis [Massenprozent] | | |
|---|---|---|---|
| CO₂/Wasser Kohlensäure | Dodecen | Thiol | Thioether |
| | 3,5 | 93,5 | 3,0 |

### Beispiel 2

Die folgende Umsetzung wird in einem gerührten Autoklaven (V = 0,3 I) durchgeführt, der mit einem Begasungsrührer, Strombrechern und einer temperierbaren Doppelmantelhülle ausgestattet ist. Dodecen und Wasser werden vor dem Verschließen des Reaktors eingefüllt. Anschließend temperiert man den Reaktorinhalt bei schnell laufendem Rührmotor auf die gewünschte Reaktionstemperatur und verdrängt die Luft enthaltende Gasphase des Reaktors durch mehrmaliges Aufpressen von 10 bar Kohlendioxids und lässt den Druck des letzten Spülvorgangs auf dem System stehen. Nun wird bei stehendem Rührmotor Schwefelwasserstoff auf den gewünschten Reaktionsdruck in das System eingepresst und unmittelbar darauf der Rührer des Autoklaven gestartet. Während des Experiments bleibt die H2S-Zuleitung des Reaktors geöffnet, so dass die abreagierte Schwefelwasserstoffmenge in das Reaktionssystem nachgeliefert wird. Während der nächsten zwei Stunden wird gleichmäßig auf 60 °C erwärmt. Nach 3 weiteren Stunden werden mittels Druckabsenkung am Entnahmehahn und zusätzliches Aufheizen auf 100 °C alle gelösten Gasbestandteile aus der zweiphasigen Flüssigprobe entfernt. Die quantitative und qualitative Auswertung dieser Experimente erfolgt durch den zeitlichen H2S-Verbrauch und gaschromatographische Analysen der Flüssigphase. Die nachfolgende Tabelle 2 stellt zwei Experimente gegenüber, die bis auf die Wasserzugabe nahezu identisch durchgeführt werden. Ohne Wasserzugabe befindet sich nur die vom Olefin mitgebrachte Wassermenge im System (ca. 50 ppm) und es findet kaum merklich eine Umsetzung der olefinischen Doppelbindung statt.

**Tabelle 2**

| Säure | Wasser [g] | Dodecen [g] | Temp. [°C] | CO₂-Druck [bar] | H₂S- Druck [bar] | Drehzahl [min⁻¹] | Zeit [min] | GC-Analysen-Ergebnis [Massenprozent] | | |
|---|---|---|---|---|---|---|---|---|---|---|
| | | | | | | | | Dodecen | Thiol | Thioether |
| CO₂ | 0 | 60 | 30-60 | 10 | 15 | 1600 | 300 | 99,7 | 0,2 | 0,1 |
| CO₂/Wasser Kohlensäure | 20 | 60 | 30-60 | 10 | 15 | 1600 | 300 | 3,9 | 93,1 | 3,0 |

## Patentansprüche

1. Verfahren zur Herstellung von schwefelhaltigen Verbindungen der allgemeinen Formel I 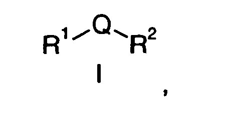 worin Q, R¹ und R² unabhängig voneinander die folgenden Bedeutungen haben:
Q: -S- oder -S-S-,
R¹: Wasserstoff oder gesättigter oder ungesättigter, linearer oder verzweigter C₁-C₃₀-Alkylrest,
R²: Wasserstoff oder gesättigter oder ungesättigter, linearer oder verzweigter C₁-C₃₀-Alkylrest,
wobei R¹ und R² nicht gleichzeitig Wasserstoff sind, durch Umsetzung eines Schwefelwasserstoff und gegebenenfalls Sauerstoff enthaltenden Mischgasstroms mit linearen oder verzweigten C₁-C₃₀-Olefinen, **dadurch gekennzeichnet, dass** die Umsetzung in Gegenwart von Wasser und Kohlendioxid bei einem Druck von 2 bis 325 bar durchgeführt wird.

2. Verfahren nach Anspruch 1, **dadurch gekennzeichnet, dass** Wasser in der Reaktionsmischung zu 0,1 bis 50 Gew.-% vorliegt.

3. Verfahren nach Anspruch 1 oder 2, **dadurch gekennzeichnet, dass** es bei einem Druck von 10 bis 100 bar durchgeführt wird.

4. Verfahren nach einem der Ansprüche 1 bis 3, **dadurch gekennzeichnet, dass** R¹ und R² unabhängig voneinander Wasserstoff oder gesättigter oder ungesättigter, linearer oder verzweigter C₈-C₂₂-Alkylrest bedeuten, wobei R¹ und R² nicht gleichzeitig Wasserstoff sind.

5. Verfahren nach einem der Ansprüche 1 bis 4, **dadurch gekennzeichnet, dass** R¹ und R² unabhängig voneinander Wasserstoff oder einen gesättigten Alkylrest mit 12 Kohlenstoffatomen bedeuten, wobei R¹ und R² nicht gleichzeitig Wasserstoff bedeuten.

6. Verfahren nach einem der Ansprüche 1 bis 5, **dadurch gekennzeichnet, dass** Q in Formel (I) -S-S- bedeutet, wenn Sauerstoff in dem Mischgasstrom enthalten ist.

7. Verfahren nach einem der Ansprüche 1 bis 5, **dadurch gekennzeichnet, dass** aus Dodecen tert.-Dodecylthiol hergestellt wird.

8. Verfahren nach einem der Ansprüche 1 bis 7, **dadurch gekennzeichnet, dass** das Mischgas aus dem Abgasstrom eines chemischen Verfahrens stammt.

## Claims

1. A process for preparing sulfur-comprising compounds of the general formula I 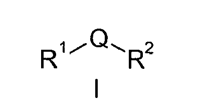 in which Q, R¹ and R² are each independently defined as follows:
Q: -S- or -S-S-,
R¹: hydrogen or saturated or unsaturated, linear or branched C₁-C₃₀-alkyl radical,
R²: hydrogen or saturated or unsaturated, linear or branched C₁-C₃₀-alkyl radical,
where R¹ and R² are not simultaneously hydrogen, by reacting a mixed gas stream comprising hydrogen sulfide, with or without oxygen, with linear or branched C₁-C₃₀-Olefins, which comprises carrying out the reaction in the presence of water and carbon dioxide at a pressure of from 2 to 325 bar.

2. The process according to claim 1, wherein water is present in the reaction mixture to an extent of from 0.1 to 50% by weight.

3. The process according to claim 1 or 2, which is carried out at a pressure of from 10 to 100 bar.

4. The process according to any of claims 1 to 3, wherein R¹ and R² are each independently hydrogen or saturated or unsaturated, linear or branched C₈-C₂₂-alkyl radical, where R¹ and R² are not simultaneously hydrogen.

5. The process according to any of claims 1 to 4, wherein R¹ and R² are each independently hydrogen or a saturated alkyl radical having 12 carbon atoms, where R¹ and R² are not simultaneously hydrogen.

6. The process according to any of claims 1 to 5, wherein Q in formula (I) is -S-S- when oxygen is present in the mixed gas stream.

7. The process according to any of claims 1 to 5, wherein tert-dodecyl thiol is prepared from dodecene.

8. The process according to any of claims 1 to 7, wherein the mixed gas stems from the offgas stream of a chemical process.

## Revendications

1. Procédé de préparation de composés sulfurés de formule générale I 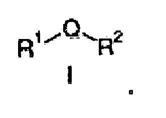 dans laquelle Q, R¹ et R² ont, indépendamment les uns des autres, les caractéristiques suivantes:
Q : -S- ou -S-S-,
R¹: hydrogène, ou résidu alkyle en C₁-C₃₀ linéaire ou ramifié, saturé ou insaturé,
R² : hydrogène, ou résidu alkyle en C₁-C₃₀ linéaire ou ramifié, saturé ou insaturé,
où R¹ et R² ne sont pas simultanément hydrogène, par conversion d'un flux de gaz mélangé contenant de l'hydrogène sulfuré et éventuellement de l'oxygène avec des oléfines en C₁-C₃₀ linéaires ou ramifiés, **caractérisé en ce que** la conversion est réalisée en présence d'eau et de dioxyde de carbone à une pression de 2 à 325 bars.

2. Procédé selon la revendication 1, **caractérisé en ce que** l'eau représente de 0,1 à 50 % en poids du mélange de la réaction.

3. Procédé selon la revendication 1 ou 2, **caractérisé en ce qu'**il est réalisé à une pression de 10 à 100 bars.

4. Procédé selon l'une quelconque des revendications 1 à 3, **caractérisé en ce que** R¹ et R² désignent indépendamment l'un de l'autre un hydrogène ou un résidu alkyle en C₈-C₂₂ linéaire ou ramifié, saturé ou insaturé, où R¹ et R² ne sont pas simultanément hydrogène.

5. Procédé selon l'une quelconque des revendications 1 à 4, **caractérisé en ce que** R¹ et R² désignent indépendamment l'un de l'autre un hydrogène ou un résidu alkyle saturé comportant 12 atomes de carbone, où R¹ et R² ne sont pas simultanément hydrogène.

6. Procédé selon l'une quelconque des revendications 1 à 5, **caractérisé en ce que** Q dans la formule (I) désigne -S-S- si le flux de gaz mélangé contient de l'oxygène.

7. Procédé selon l'une quelconque des revendications 1 à 5, **caractérisé en ce qu'**on réalise du dodécylthiol tertiaire à partir de dodécène.

8. Procédé selon l'une quelconque des revendications 1 à 7, **caractérisé en ce que** le gaz mélangé provient du flux de gaz résiduel d'un procédé chimique.
